# EUROPÄISCHE PATENTANMELDUNG

(11) **EP 4 461 701 A1**
(43) Veröffentlichungstag der Anmeldung: **13.11.2024**
(21) Anmeldenummer: 23020220.2
(22) Anmeldetag: 12.05.2023
(51) Int. Cl.: C01B 3/38, G01N 33/22

(54) **VERFAHREN UND ANLAGE ZUR ERZEUGUNG EINES ODER MEHRERER VERFAHRENSPRODUKTE**

(71) Anmelder: Linde GmbH, 82049 Pullach (DE)
(72) Erfinder: Schwarzhuber, Josef, 82049 Pullach (DE); Helfenbein, Sebastian, 82049 Pullach (DE)
(74) Vertreter: Fischer, Werner

(57) **Zusammenfassung**

Es wird ein Verfahren sowie eine Vorrichtung zur Erzeugung eines Verfahrensprodukts, bei dem ein Einsatzgemisch unter Erhalt eines gasförmigen, das Verfahrensprodukt enthaltenden Produktgemisches (3, 4) in einem über einen Brenner (B) beheizten Reaktionsraum (R) eines Reaktors (100) umgesetzt wird. Kennzeichnend hierbei ist, dass in einem ersten Verfahrensmodus der Brenner (B) mit einem ersten Brenngas (911) betrieben wird und in einem zweiten Verfahrensmodus ein zur Verfügung gestelltes brennbaren Gas (921) mit einem Zusatzgas (P, X) gemischt wird, um ein zweites zum Betrieb des Brenners (B) geeignetes Brenngas zu erzeugen.

## Beschreibung

Die vorgeschlagene Erfindung betrifft ein Verfahren und Anlage zur Erzeugung eines Verfahrensprodukts.

### Hintergrund

Bei einer Vielzahl von Verfahren zur Herstellung chemischer Verfahrensprodukte werden Reaktoren eingesetzt, die durch Brenner beheizte Reaktionsräume umfassen, durch welche ein Einsatz geführt und dabei zumindest teilweise zu den gewünschten Reaktionsprodukten umgesetzt wird. Beispiele für entsprechende Verfahren sind das Dampfspalten (Steamcracking), die Dehydrierung von Alkanen oder auch die Synthesegaserzeugung, insbesondere unter Einsatz unterschiedlicher Reformierungsverfahren wie der Dampfreformierung.

Die Dampfreformierung ist seit langem bekannt und beispielsweise im Artikel "Gas Production, 2. Processes" in Ullmann's Encyclopedia of Industrial Chemistry, Onlineausgabe 15. Oktober 2011, doi: 10.1002/14356007.o12_o01, beschrieben. Die vorliegende Erfindung und ihre Ausgestaltungen werden nachfolgend überwiegend unter Bezugnahme auf die Dampfreformierung beschrieben, sind aber nicht auf einen entsprechenden Einsatz beschränkt, sondern eignen sich insbesondere auch für die erwähnten anderen Verfahren, in denen entsprechende Brenner eingesetzt werden.

Bei der Dampfreformierung wird ein kohlenwasserstoff- und dampfhaltiges Einsatzgemisch parallel durch eine Vielzahl von typischerweise vertikal verlaufenden, über Brenner beheizte Reaktionsrohren geführt, die insgesamt einen Reaktionsraum bilden und in einem Reaktorbehälter angeordnet sind. In den Reaktionsrohren ist ein für die Dampfreformierung geeigneter Katalysator auf einem entsprechenden Trägermaterial bereitgestellt. Das Einsatzgemisch wird über Verteiler(rohre) auf die Reaktionsrohre verteilt und ein durch die Dampfreformierung erhaltenes Produktgemisch wird über Sammler(rohre) zusammengefasst und abgeleitet.

Die Erfindung bezieht sich insbesondere auf den Betrieb von Reformerbrennern, die für Erdgas als Brenngas ausgelegt sind, ist aber ebenfalls nicht hierauf beschränkt. Zur Optimierung der Wirtschaftlichkeit von Reformern wird von diesen immer häufiger Flexibilität bezüglich der Einsatzstoffe gefordert. Entsprechendes gilt auch für andere Anlagen, Reaktoren bzw. Brenner. Insbesondere der vollständige oder teilweise Ersatz von Erdgas durch schwerere Kohlenwasserstoffe wie Propan, Butan und anderen wird gefordert, da diese als Brenngas, aber auch als Einsatz wirtschaftlich und ggf. hinsichtlich ihrer Eigenschaften vorteilhaft sein können.

Wenn eine entsprechende Gesamtanlage auf ein anderes brennbares Gas für den Einsatz in den Brennern umgestellt werden soll (aufgrund von Wirtschaftlichkeit, Verfügbarkeit und dergleichen), müssen die Brenner auf die Eignung für den Betrieb mit dem neuen brennbaren Gas untersucht werden. Es besteht normalerweise die Forderung, die Anlage sowohl mit den bisherigen Brenngasen als auch mit den neuen, in der Regel schwerere Komponenten umfassenden brennbaren Gas stabil und effizient betreiben zu können.

Es werden somit deutlich höhere Anforderungen an die Brennerflexibilität gestellt. Im Normalfall dürfen sich die Brenngase nur wenig voneinander unterscheiden, da sich sonst die Bedingungen an den Brennerdüsen so stark ändern würden, dass das Flammenbild nicht mehr akzeptabel ist oder die Emissionen das erlaubte Maß überschreiten.

Im hier betrachteten Fall sollen die Brenner beispielsweise anstatt mit Erdgas mit Propan betrieben werden, wobei ein reiner Erdgasbetrieb auch weiterhin möglich sein muss. Propan weist eine wesentlich höhere Wobbezahl als Erdgas auf und kann nicht ohne weitere Maßnahmen in einem Erdgas-Brenner verwendet werden, da sich dabei ein sehr schlechtes und nicht akzeptables Flammenbild ergibt. Auch die Emissionswerte werden sich wahrscheinlich über das akzeptable Maß erhöhen. Wenn das Propan nicht sauber verbrennt, könnte sich außerdem Kohlenstoff (Koks) in oder an den Brennerdüsen bilden, was zu einer weiteren Verschlechterung des Flammenbildes führt und auch eine unerwünschte Ungleichverteilung der Flammen zur Folge haben kann, die u.U. mit weiteren Schäden am Brenner oder am Reaktor einhergeht.

Es sind daher Maßnahmen wünschenswert, die es ermöglichen, entsprechende Brenner mit unterschiedlichen Brenngasen flexibel betreiben zu können. Zusätzliche Investitionskosten sollen minimiert werden.

### Offenbarung der Erfindung

Vor diesem Hintergrund werden ein Verfahren und eine Anlage mit den Merkmalen der unabhängigen Patentansprüche vorgeschlagen. Ausgestaltungen der Erfindung sind in den abhängigen Patentansprüchen und der nachfolgenden Beschreibung angegeben.

Das vorgeschlagene Verfahren zur Erzeugung eines Verfahrensprodukts umfasst, dass ein Einsatzstoff unter Erhalt eines gasförmigen, das Verfahrensprodukt enthaltenden Produktgemisches in einem über einen Brenner beheizten Reaktionsraum eines Reaktors umgesetzt wird, wobei in einem ersten Verfahrensmodus der Brenner mit einem ersten Brenngas betrieben wird und in einem zweiten Verfahrensmodus ein zur Verfügung gestelltes brennbaren Gas mit einem Zusatzgas gemischt wird, um ein zweites zum Betrieb des Brenners geeignetes Brenngas zu erzeugen.

Durch die Zumischung des Zusatzgases können die Eigenschaften des im zweiten Verfahrensmodus zur Verfügung gestellten brennbaren Gases vorteilhaft so beeinflusst werden, dass das brennbare Gas, auf dessen Einsatz der Brenner originär nicht ausgelegt sein muss, als Brenngas eingesetzt werden kann. Die Eigenschaften des zweiten Brenngases werden so eingestellt, dass sich an der oder den Brennerdüsen des Brenners Bedingungen einstellen, unter denen der Brenner betreibbar ist. Mit der vorgeschlagenen Lösung ist damit insbesondere eine reversible Nachrüstung bzw. Erweiterung (engl. Revamp) einer entsprechenden Anlage möglich, die damit flexibel betrieben werden kann.

Das Zusatzgas kann insbesondere ein Anteil des Produktgemischs sein oder einen Anteil des Produktgemischs umfassen. In einer derartigen Ausgestaltung kann auf extern bereitgestellte Gase zur Erzeugung des zweiten Brenngases u.U. verzichtet werden.

Ein entsprechender Anteil des Produktgemischs kann von dem Produktgemisch abgezweigt oder abgetrennt werden, wobei unter "abzweigen" hier insbesondere eine Entnahme bzw. Ableitung einer Teilmenge in unveränderter Zusammensetzung verstanden werden soll, und unter "abtrennen" die Bildung einer Fraktion mit geänderter Zusammensetzung in einer geeigneten Trenneinrichtung. Beispielsweise kann eine entsprechende Einrichtung eine Druckwechseladsorptionsanlage aufweisen und eine entsprechende Abzweigung oder Abtrennung kann in oder stromauf dieser Druckwechseladsorptionsanlage erfolgen.

Alternativ oder zusätzlich kann das Zusatzgas ein extern bereitgestelltes Gas oder Gasgemisch sein oder ein solches umfassen. In einer derartigen Ausgestaltung kann durch die unabhängige Wahl des Zusatzgases und dessen Eigenschaften insbesondere eine zielgerichtete Beeinflussung der Eigenschaften des zweiten Brenngases erfolgen.

Das Zusatzgas kann aus einem oder mehreren brennbaren Gasen, einem oder mehreren Inertgasen oder Mischungen hiervon ausgewählt sein. Bei einem brennbaren Gas kann es sich beispielsweise um Wasserstoff, Methan oder einen höheren Kohlenwasserstoff handeln. Unter einem "Inertgas" soll vorliegend nicht lediglich ein klassisches, als inert bezeichnetes Gas wie Stickstoff oder ein Edelgas verstanden werden, sondern beispielsweise auch ein Gas wie Kohlendioxid, das sich in dem Brenner inert verhält, d.h. insbesondere nicht verbrennt.

In sämtlichen Ausgestaltungen des vorgeschlagenen Verfahrens kann insbesondere Luft als das oder als ein Teil des Zusatzgases verwendet werden, beispielsweise auch sauerstoffangereicherte Luft. Auf diese Weise kann ebenfalls auf die Verbrennungscharakteristik des zweiten Brenngases Einfluss genommen werden.

In Ausgestaltungen der Erfindung weist das erste Brenngas eine Wobbezahl auf, die von einer Wobbezahl des im zweiten Verfahrensmodus zur Verfügung gestellten brenngaren Gases abweicht, wobei die Wobbezahl des brennbaren Gases durch die Zumischung eines oder mehrerer Zusatzgase an die Wobbezahl des ersten Brenngases zumindest angeglichen wird. Eine vollständige Übereinstimmung der beiden Wobbezahlen muss dabei nicht erreicht werden; Abweichungen von bis zu 1% oder 5% oder 10% sind zulässig.

Eine entsprechende Ausgestaltung sieht insbesondere die Verwendung einer Steuereinheit vor, die die Wobbezahl des zweiten Brenngases überwacht und die zuzumischende Menge des oder der Zusatzgase vorgibt. Auf diese Weise kann rasch auf Schwankungen in der Zusammensetzung des zur Verfügung gestellten brennbaren Gases reagiert werden, wie sie beispielsweise auftreten können, wenn das brennbare Gas aus Gasen gemischt wird, die unterschiedlichen Quellen entstammen.

Insbesondere kann das erste Brenngas ein methanreiches Gas oder Gasgemisch sein oder umfassen, und/oder das für den zweiten Verfahrensmodus zur Verfügung gestellte brennbare Gas kann ein überwiegend oder ausschließlich Kohlenwasserstoffe mit zwei oder mehr Kohlenstoffatomen enthaltendes Gas oder Gasgemisch sein oder umfassen. Entsprechende Ausgestaltungen ermöglichen damit die Nutzung unterschiedlicher Gase bzw. Gasgemische, beispielsweise je nach Angebot und/oder Kosten.

Das erste Brenngas kann in entsprechenden Ausgestaltungen insbesondere Erdgas, Biogas oder Vergasungsgas, z.B. aus der Vergasung von festen oder flüssigen Einsatzstoffen wie Kohle, Abfall, Biomasse oder dergleichen sein oder umfassen. Das brennbare Gas des zweiten Verfahrensmodus kann Propan sein oder umfassen.

Wie erwähnt, eignet sich das hier vorgeschlagene Verfahren für unterschiedliche Umsetzungen, so dass der Reaktor in entsprechenden Ausgestaltungen zur Dampfreformierung, zur autothermen Reformierung, zum Dampfspalten, für eine katalytische Dehydrierung oder eine katalytische Oxidation eingerichtet sein kann. Das vorgeschlagene Verfahren hat damit eine Vielzahl unterschiedlicher Anwendungsbereiche und kann jeweils an diese angepasst werden.

In dem hier vorgeschlagenen Verfahren und entsprechenden Ausgestaltungen kann das Zusatzgas stromauf des Brenners zur Erzeugung des zweiten Brenngases dem brennbaren Gas zugemischt werden. Auf diese Weise können besonders homogene Eigenschaften des zweiten Brenngases erreicht werden.

In Ausgestaltungen der vorliegenden Erfindung können das erste und das zweite Brenngas dem Brenner insbesondere über eine oder mehrere erste Brennerlanzen zugeführt werden. Dem Brenner kann ferner das erste Brenngas, das zweite Brenngas oder wenigstens ein beliebiges weiteres Brenngas über zumindest eine weitere Brennerlanze zugeführt werden. Die Erfindung ist anwendbar auf jede der Brenngaslanzen zu einem entsprechenden Brenner. Aspekte von entsprechenden Ausgestaltungen der Erfindung können dabei eine Reduktion der Anzahl notwendiger Lanzen bei neuen Brennern und/oder die Verwendung bestehender Lanzen mit mehr Brenngasen als zuvor bei bereits bestehenden Brennern umfassen und entsprechende Nachrüstvorteile bieten.

Die vorgeschlagene Anlage zur Erzeugung eines Verfahrensproduktes umfasst einen Reaktor mit einem Reaktionsraum und einem Brenner zur Beheizung des Reaktionsraums, der eine Zuführeinrichtung für die Zuführung eines Einsatzgemisches sowie eine Abzugseinrichtung für den Abzug eines im Reaktionsraum erhältlichen, zumindest ein Verfahrensprodukt enthaltenden gasförmigen Produktgemisches aufweist. Die Anlage, die dafür eingerichtet ist, den Brenner in einem ersten Verfahrensmodus unter Verwendung eines ersten Brenngases zu betreiben, weist eine Mischeinrichtung auf, über die in einem zweiten Verfahrensmodus ein brennbares Gas mit einem Zusatzgas gemischt werden kann, um ein zweites Brenngas für den Betrieb des Brenners zu erzeugen.

Zu weiteren Merkmalen und Vorteilen einer entsprechenden Anlage und Ausgestaltungen hiervon sei auf die obigen Erläuterungen betreffend das erfindungsgemäß vorgeschlagene Verfahren und seine Ausgestaltungen ausdrücklich verwiesen, da diese hierfür in gleicher Weise gelten.

Entsprechendes gilt auch für eine Anlage, die gemäß einer Ausgestaltung der Erfindung dazu eingerichtet ist, ein Verfahren gemäß einer beliebigen Ausgestaltung der vorliegenden Erfindung durchzuführen.

### Kurze Beschreibung der Zeichnung

Ausführungsformen der Erfindung werden nachfolgend rein beispielhaft unter Bezugnahme auf die beigefügte Zeichnung unter Erläuterung des technischen Hintergrunds beschrieben.
Figur 1 veranschaulicht einen Reaktor gemäß einer Ausführungsform der Erfindung in vereinfachter, schematischer Darstellung.
Figur 2 veranschaulicht einen Reaktor gemäß einer Ausführungsform der Erfindung in vereinfachter, schematischer Teildarstellung.

### Ausführungsformen der Erfindung

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar, und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

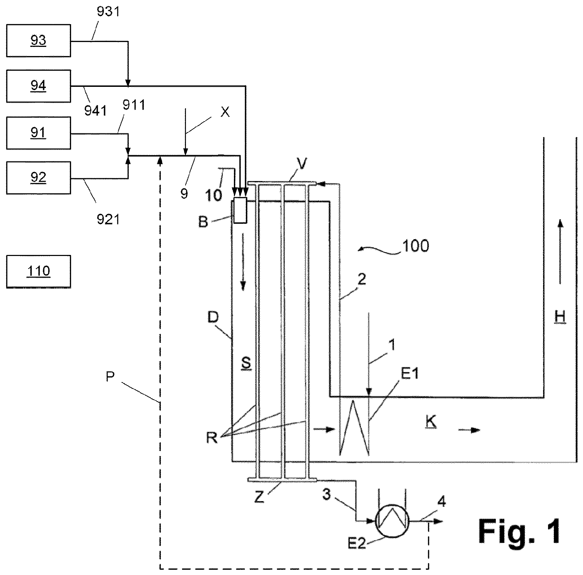

In Figur 1 ist ein Reaktor 100 zur Dampfreformierung zur Veranschaulichung einer Ausgestaltung der Erfindung stark vereinfacht dargestellt.

Der hier veranschaulichte Reaktor 100 kann insbesondere Teil einer Synthesegasanlage sein, in der aus einem kohlenwasserstoff- und wasserdampfhaltigen Einsatzgemisch, das beispielsweise Erdgas umfasst, ein Synthesegas an sich bekannter Art erzeugt wird. Das Synthesegas stellt dabei das in dem dargestellten Beispiel erzeugte Produktgemisch dar.

Über eine Leitung 1 wird das Einsatzgemisch in einen Wärmetauscher E1, der in einer Konvektionszone K angeordnet ist, eingeleitet, und gegen heißes Rauchgas vorgewärmt. Die Konvektionszone K ist Teil eines Rauchgassystems. Das vorgewärmte Einsatzgemisch wird über eine Leitung 2 zu einem Verteiler V geführt und auf die mit einem geeigneten Katalysatormaterial gefüllten, insgesamt einen Reaktionsraum bildenden Reaktionsrohre R verteilt, in denen es in einer endothermen Reaktion in das Synthesegas umgesetzt wird.

Das Synthesegas wird in einem Sammler Z gesammelt und über eine Leitung 3 einem Kühlersystem E2 zugeleitet. In dem Kühlersystem E2 wird das Synthesegas bis unterhalb des Wassertaupunkts abgekühlt, wobei eine Gasfraktion 4 und nicht gesondert veranschaulichtes Prozesskondensat erzeugt werden. Zu weiteren Details bezüglich der Behandlung der Gasfraktion 4 sei, wie bereits mehrfach zuvor, auf die einschlägige Fachliteratur verwiesen.

Die für die endotherme Dampfreformierungsreaktion benötigte Energie wird über Brenner B erzeugt, von denen einer in Figur 1 sehr stark vereinfacht dargestellt ist, und in denen ein Brenngasstrom 9 mit Luft 10 unter Sauerstoffüberschuss verbrannt wird. Das dabei erzeugte heiße Rauchgas, dessen Strömungsrichtung durch Pfeile angedeutet ist, gibt einen großen Teil seiner fühlbaren Wärme vorwiegend durch Strahlung in der Strahlungszone S an die durch die Reaktionsrohre R strömenden Reaktionspartner in dem Einsatzgemisch ab.

Mit hoher Temperatur wird das teilweise abgekühlte Rauchgas aus der Strahlungszone S abgezogen und in die Konvektionszone K eingeleitet, in der es gegen anzuwärmende Verfahrensströme, unter anderem das Einsatzgemisch 1, abgekühlt wird, bevor es über den Kamin H und eventuell entsprechende Reinigungseinrichtungen in die Atmosphäre gelangt.

Der Brenngasstrom 9 kann in dem dargestellten Beispiel wahlweise entweder unter Verwendung einer ersten Brenngasversorgungseinrichtung 91 oder unter Verwendung einer zweiten Brenngasversorgungseinrichtung 92 bereitgestellt werden. Beispielsweise kann es sich dabei, unabhängig voneinander, jeweils um ein Versorgungsnetz, eine Versorgungsleitung oder einen Versorgungstank oder beliebige Kombinationen oder Ausgestaltungen entsprechender Einrichtungen handeln. Eine Bereitstellung entsprechender Brenngase im gleichen Aggregatzustand oder in unterschiedlichen Aggregatzuständen kann vorgesehen sein.

Die erste Brenngasversorgungseinrichtung 91 und die zweite Brenngasversorgungseinrichtung 92 sind dafür eingerichtet, unterschiedliche Brenngase bereitzustellen, und zwar mindestens ein erstes Brenngas 911 und ein zweites Brenngas 921. Beispielsweise kann die erste Brenngasversorgungseinrichtung 91 zur Bereitstellung von Erdgas als erstem Brenngas 911 und die zweite Brenngasversorgungseinrichtung 92 zur Bereitstellung von Propan als zweitem Brenngas 921 eingerichtet sein. Es versteht sich, dass auch eine gemeinsame Brenngasversorgungseinrichtung zur Bereitstellung unterschiedlicher Brenngase eingerichtet sein kann.

Eine Steuereinheit 110 ist bereitgestellt, die zum Umschalten zwischen zumindest zwei Verfahrensmodi eingerichtet ist. Ein erster Verfahrensmodus umfasst die Verwendung des ersten Brenngases 911 in dem Brenner B, insbesondere aus der ersten Brenngasversorgungseinrichtung 91, und ein zweiter Verfahrensmodus die Verwendung eines brennbaren Gases 921, insbesondere aus der zweiten Brenngasversorgungseinrichtung 92.

In dem zweiten Betriebsmodus wird in den Brenner B ein Zusatzgas eingespeist, insbesondere ein Produktrecyclestrom P, der in dem dargestellten Beispiel von der Gasfraktion 4 abgetrennt oder abgezweigt werden kann. Beliebige Aufbereitungsschritte können vorgesehen sein, sind aber der Übersichtlichkeit nicht gesondert veranschaulicht. Der Produktrecyclestrom P wird in dem dargestellten Beispiel stromauf des Brenners B mit dem zweiten Brenngas 921 gemischt und als zweites Brenngas dem Brenner B zugeführt.

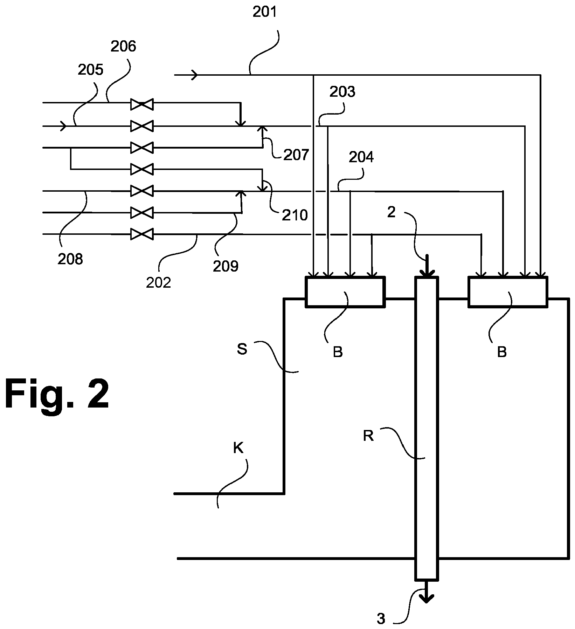

Der Brenner B kann bereits zwei unterschiedliche Gase parallel verbrennen, wie auch nachfolgend in Figur 2 veranschaulicht, die aber über zwei unterschiedliche Brennerlanzen zum Brenner B geführt werden können. Erdgas kann insbesondere dann vorteilhaft durch Propan, das über dieselbe Brennerlanze geführt wird, ersetzt werden, wenn Synthesegas zugemischt und auf diese Weise die Wobbezahl des zweiten Brenngases angepasst wird.

Alternativ oder zusätzlich zur Verwendung des Produktrecyclestroms P kann auch eine entsprechende Verwendung eines oder mehrerer weiterer Gase oder Gasgemische X erfolgen, wie bereits zuvor erläutert. In dem oder den Brennern können auch noch über separate Brenngaszuführungen und Brenngaslanzen zusätzliche Brenngase verbrannt werden, wie insbesondere anhand der nachfolgenden Figur 2 erläutert wird. Dies ist in dem in Figur 1 dargestellten Beispiel anhand weiterer Gasversorgungseinrichtungen 93, 94 bzw. entsprechender Brenngase 931, 941 veranschaulicht. Es versteht sich, dass entsprechende Brenngase auch aus gemeinsamen Gasversorgungseinrichtungen entnommen werden können und zum Teil identische Brenngase sein können.

In Figur 2 ist ein Reaktor gemäß einer weiteren Ausführungsform der Erfindung in vereinfachter, schematischer Teildarstellung veranschaulicht, wobei die bereits in Figur 1 verwendeten Bezugszeichen teilweise weiterverwendet werden und die obigen Erläuterungen hierfür jeweils weiter gelten.

Abweichend zu dem in Figur 1 veranschaulichten Reaktor sind die Reaktionsrohre R vereinfacht dargestellt und zwei Brenner B veranschaulicht. Diesen Brennern wird über eine sich entsprechend verzweigende Leitung 201 jeweils Verbrennungsluft zugeführt. Ferner wird den Brennern B über eine Leitung 202 ein Brenngas, beispielsweise Erdgas, zugeführt, um Pilotbrenner entsprechen betreiben zu können.

Die Brenner können über gemeinsame oder gesonderte Brennerlanzen über Leitungen 203 und 204 mit Niederdruckbrenngas bzw. Hochdruckbrenngas oder allgemeiner einem ersten und einem zweiten Brenngasgemisch versorgt werden. Das Niederdruckbrenngas in Leitung 203 kann insbesondere unter Verwendung von Spülgas aus einer Druckwechseladsorption gebildet werden, das über Leitung 205 bereitgestellt wird. Diesem Spülgas kann über eine Leitung 206 ein kohlendioxidreiches Abgas aus einer entsprechenden Druckwechseladsorption und über eine Leitung 207 Synthesegas zugegeben werden.

Das Hochdruckbrenngas in Leitung 204 kann insbesondere unter Verwendung von Erdgas gebildet werden, das über eine Leitung 208 bereitgestellt wird. Diesem kann über eine Leitung 209 Propan und über eine Leitung 210 wiederum Synthesegas zugegeben werden.

Wie bereits zuvor angesprochen, verbrennen die Brenner B hier bereits zwei verschiedene Brenngasströme, die über die Leitungen 203 bzw. 204 und entsprechende Brennerlanzen parallel zugeführt werden. Das Erdgas 208 kann dann durch Propan aus Leitung 209 ersetzt werden, geführt über die gleiche Leitung 204 bzw. die gleiche Brennerlanze, wenn das Synthesegas aus Leitung 210 dazugemischt, und so der die Wobbezahl in der Leitung 204 angepasst wird.

## Patentansprüche

1. Verfahren zur Erzeugung eines Verfahrensprodukts, bei dem ein Einsatzgemisch (1, 2) unter Erhalt eines gasförmigen, das Verfahrensprodukt enthaltenden Produktgemisches (3, 4) in einem über einen Brenner (B) beheizten Reaktionsraum (R) eines Reaktors (100) umgesetzt wird, **dadurch gekennzeichnet, dass** in einem ersten Verfahrensmodus der Brenner (B) mit einem ersten Brenngas (911) betrieben wird und in einem zweiten Verfahrensmodus ein zur Verfügung gestelltes brennbaren Gas (921) mit einem Zusatzgas (P, X) gemischt wird, um ein zweites zum Betrieb des Brenners (B) geeignetes Brenngas zu erzeugen.

2. Verfahren nach Anspruch 1, bei dem das Zusatzgas (P, X) ein Anteil des Produktgemischs (3, 4) ist oder einen Anteil des Produktgemischs (3, 4) umfasst.

3. Verfahren nach Anspruch 2, bei dem der Anteil des Produktgemischs (3, 4) von dem Produktgemisch abgezweigt oder abgetrennt wird.

4. Verfahren nach einem der vorstehenden Ansprüche, bei dem das Zusatzgas (P, X) ein unabhängig von dem Produktgemisch (3, 4) und/oder extern des Verfahrens bereitgestelltes weiteres Gas oder Gasgemisch (X) ist oder ein solches umfasst.

5. Verfahren nach Anspruch 4, bei dem das weitere Gas oder Gasgemisch (X) aus einem oder mehreren brennbaren Gasen, einem oder mehreren Inertgasen oder Mischungen hiervon ausgewählt ist.

6. Verfahren nach einem der vorstehenden Ansprüche, bei dem das erste Brenngas (911) eine Wobbezahl aufweist, die von der Wobbezahl des im zweiten Verfahrensmodus zur Verfügung gestellten brenngaren Gases (921) abweicht, wobei die Wobbezahl des brennbaren Gases (921) durch die Zumischung eines oder mehrerer Zusatzgase (P, X) an die Wobbezahl des ersten Brenngases angeglichen wird.

7. Verfahren nach Anspruch 6, bei dem unter Verwendung einer Steuereinheit (110) die Wobbezahl des zweiten Brenngases überwacht und die zuzumischende Menge des oder der Zusatzgase (P, X) vorgegeben wird.

8. Verfahren nach einem der vorstehenden Ansprüche, bei dem das erste Brenngas (911) ein methanreiches Gas oder Gasgemisch ist oder umfasst, und bei dem das brennbare Gas (921) ein überwiegend oder ausschließlich Kohlenwasserstoffe mit zwei oder mehr Kohlenstoffatomen enthaltendes Gas oder Gasgemisch ist oder umfasst.

9. Verfahren nach Anspruch 8, bei dem das erste Brenngas (911) Erdgas, Biogas oder Vergasungsgas ist oder umfasst und das brennbare Gas (921) Propan ist oder umfasst.

10. Verfahren nach einem der vorstehenden Ansprüche, bei dem der Reaktor (100) zur Dampfreformierung, zur autothermen Reformierung, zum Dampfspalten, für eine katalytische Dehydrierung oder eine katalytische Oxidation eingerichtet ist.

11. Verfahren nach einem der vorstehenden Ansprüche, bei dem das Zusatzgas (P, X) stromauf des Brenners (B) dem brennbaren Gas (921) zugemischt wird.

12. Anlage zur Erzeugung eines Verfahrensproduktes, umfassen einen Reaktor (100) mit einem Reaktionsraum (R) und einem Brenner (B) zur Beheizung des Reaktionsraums (R), der eine Zuführeinrichtung (V) für die Zuführung eines Einsatzgemisches (1, 2) sowie eine Abzugseinrichtung (Z) für den Abzug eines im Reaktionsraum (R) erhältlichen, zumindest ein Verfahrensprodukt enthaltenden gasförmigen Produktgemisches (3, 4) aufweist, **dadurch gekennzeichnet, dass** die Anlage, die dafür eingerichtet ist, den Brenner (B) in einem ersten Verfahrensmodus unter Verwendung eines ersten Brenngases (911) zu betreiben, eine Mischeinrichtung (9) aufweist, über die in einem zweiten Verfahrensmodus ein brennbares Gas (921) mit einem Zusatzgas (P, X) gemischt werden kann, um ein zweites Brenngas für den Betrieb des Brenners (B) zu erzeugen.

13. Anlage nach Anspruch 14, die zur Durchführung eines Verfahrens nach einem der Ansprüche 1 bis 11 eingerichtet ist.
